# EUROPEAN PATENT APPLICATION

(11) **EP 4 123 658 A1**
(43) Date of publication of application: **25.01.2023**
(21) Application number: 21186690.0
(22) Date of filing: 20.07.2021
(51) Int. Cl.: G16H 20/40, G16H 30/40, G16H 40/63, A61B 1/00, A61B 6/00, A61B 34/20, A61B 90/00

(54) **MEDICAL VIDEO ANNOTATION USING OBJECT DETECTION AND ACTIVITY ESTIMATION**

(71) Applicant: Leica Instruments (Singapore) Pte. Ltd., Singapore 608924 (SG)
(72) Inventor: LECK, Kheng Swee, 099310 Singapore (SG); HUANG, Jie, 767999 Singapore (SG)
(74) Representative: 2SPL Patentanwälte PartG mbB

(57) **Abstract**

An apparatus configured to annotate a video of a surgical procedure is disclosed. The annotation can be based on at least one of a command executed during the surgical procedure, an instrument setting, or the video.

## Description

### Technical field

Examples relate to a method and apparatus of recording a surgical procedure.

### Background

Medical surgeries are often recorded. For example, surgeries may be filmed as a legal requirement in many countries, and as teaching resources for medical students across the world. Video records of surgeries can be generated by apparatuses that include computer systems.

### Summary

It may be desirable to improve how annotations are determined and/or added to videos of surgical procedures. Automation of annotation can reduce user fatigue from tedious repetitive processes and may aid in reducing errors in video annotations.

Herein is disclosed an apparatus configured to annotate a video of a surgical procedure based on at least one of: a command executed during the surgical procedure, an instrument setting, or the video. The apparatus can include a processor and/or a memory. The annotation method may reduce errors in the annotation and/or reduce the burden on user(s) to manually make annotations.

The command can be for determining/setting at least one of a plurality of settings of a surgical instrument, such as an optical surgical instrument. The optical surgical instrument can be configured for procedures carried out on the human eye. It is possible to reduce patient trauma by enabling faster procedures. Allowing annotations based on commands can increase the accuracy of the determined annotations. Basing the annotation on a command may allow determinations of annotations with minimal or no user intervention. Having accurate annotations can aid in the pedagogical value of the annotated video and/or reduce the risk of failing to meet legal obligations of documentation of the procedure.

The command can be for setting at least one of a plurality of settings of a surgical apparatus and/or optical instrument, such as a surgical microscope. Allowing the basis of the determination of the annotation to include commands for settings of a surgical apparatus and/or optical instrument, such as a surgical microscope can increase the accuracy of the annotations. The command can be for setting optical settings.

The optical settings can include at least one of illumination intensity, focus, magnification, illumination source, color filter (e.g. color filter selection). Alternatively/additionally, a command can be a command to start recording, which may also form at least a partial basis for the annotation. Allowing the basis of the determination of the annotation to include optical settings can increase the accuracy of the annotations. It is desirable to have a 'basis set' of commands/settings that can aid in accurately annotating the video, particularly when a machine learning algorithm is used.

The command can be at least one of a plurality of surgical commands of a surgical instrument, such as a surgical microscope. Allowing the basis of the determination of the annotation to include surgical commands can increase the accuracy of the annotations.

The surgical commands can be for ultrasound activation, ultrasound deactivation, pump activation, pump deactivation, injection, and/or suction. Allowing the basis of the determination of the annotation to include particular surgical commands can increase the accuracy of the annotations.

The determination of the annotation can be based on at least one of: an image parameter of the video, an object in the video, relative positions of at least two objects in the video, or a movement in the video. Allowing the basis of the determination of the annotation to include particular parameters, objects, relative positions, and/or movement can increase the accuracy of the annotations.

The object can be one or more identifiable objects such as at least one surgical instrument (such as a blade or phacoemulsifier) or at least one anatomical structure. Allowing the basis of the determination of the annotation to include particular objects and/or movement can increase the accuracy of the annotations.

The apparatus can determine a time stamp associated with the annotation. Time stamps can improve the user experience in editing annotations and/or finding content within the video. The apparatus can include a memory which may store a library from which the annotation(s) is selected. An annotation library may standardize the annotation, which may be useful to meet legal requirements of documentation of the procedure, and/or for providing widely understood annotation(s).

The video can be annotated with more than one annotation, each annotation having an associated time stamp. Multiple annotations may aid in accurately describing multistep surgical procedures.

A machine learning algorithm can be used to determine the annotation. Machine learning algorithms may provide accurate annotations and/or reduce the burden on users for making/editing annotations. Machine learning algorithms may also adapt and incorporate new information. Machine learning may increase accuracy and/or provide more relevant/informative annotations.

The machine learning algorithm may identify at least one of the command, the image parameter, an object, or a movement; or classify at least one of the command, the image parameter, the object, or the movement. An algorithm capable of identifying and/or classifying may increase the accuracy of annotations and/or provide more relevant/informative annotations.

A method of annotating a video of a surgical procedure is disclosed herein. The method includes determining an annotation for a video of a surgical procedure, and annotating the video with the annotation. Determining the annotation is based on at least one of: a command executed during a procedure recorded by the video, an instrument setting, or a determination of at least one of: an image parameter of the video, an object in the video, or a movement in the video. Using command(s), setting(s), image parameter(s), object identification, and/or movement may increase the accuracy of annotations and/or provide more relevant/informative annotations.

A computer program with a program code for annotating a video of a surgical procedure is disclosed herein. The annotating can be based on at least one of: a command executed during the surgical procedure, an instrument parameter, or the video. Using command(s), setting(s), instrument parameter(s), or the video may increase the accuracy of annotations and/or provide more relevant/informative annotations.

### Short description of the Figures

Some examples of apparatuses and/or methods will be described in the following by way of example only, and with reference to the accompanying figures, in which
Fig. 1 illustrates a schematic illustration of a system including a computer system;
Fig. 2 illustrates a method of annotating a video;
Fig. 3 illustrates a surgery workflow;
Fig. 4 illustrates an annotated video; and
Fig. 5 illustrates a surgical procedure.

### Detailed Description

Various examples will now be described more fully with reference to the accompanying drawings in which some examples are illustrated. In the figures, the thicknesses of lines, layers and/or regions may be exaggerated for clarity.

As used herein the term "and/or" includes any and all combinations of one or more of the associated listed items and may be abbreviated as "/". For example, "irrigation/aspiration" can mean irrigation and/or aspiration. Herein, a trailing "(s)" conveys an optional plurality. For example, "frame(s)" can mean one or more frames of a video.

Although some aspects have been described in the context of an apparatus, it is clear that these aspects also represent a description of the corresponding method, where a block or device corresponds to a method step or a feature of a method step. Analogously, aspects described in the context of a method step also represent a description of a corresponding block or item or feature of a corresponding apparatus.

Annotations of the medical video clips can be useful to highlight the moments/steps in the surgery for both documentation and educational purposes.

Some surgeries follow routine procedures, and can have standard steps. For example, in cataract surgery, port incision, second incision, viscoelastic material (e.g. viscoat) application, continuous curvilinear capsulorhesis, flap creation, phacoemulsification, suction, irrigation and intraocular lens inversion can be performed sequentially, possibly with at least some of the same steps taking approximately the same time across surgeries. Thus, annotation for this type of surgery can be similar, from procedure to procedure, and therefore repetitive if done manually. Herein is disclosed an apparatus and method for annotating a video of a surgical procedure. The methods and apparatuses described herein can make the annotations of surgical procedure videos easier.

For example, some surgeries, such as neurosurgery, may take long hours to complete. Annotating a 10 hour surgery could be tedious using state of the art methods. For similar types of surgery, the trouble of having to go through a video stream to highlight important steps can be tedious, particularly when done manually. It is possible, for medical video clips, long or short, that users can go through an entire video, pausing at steps that are of interest, and manually add annotation. Generally, manual annotation of surgical videos is time consuming, repetitive and tedious. Being prone to errors, it is also possible for undesirable information loss in some cases.

In the case of short and/or standard medical surgeries, for example, cataract surgeries, the steps can be routine. Going through very similar video clips and highlighting similar or even identical steps can be repetitive work.

In the case of longer medical surgeries, for example, neuro surgeries, the videos tend to be in the range of 10 hours or longer. Going through medical video clips with such length can be tedious. It is possible that users can neglect important steps during annotation, leading to undesirable information loss. For example, a procedure may have highlights that make up only about 1% of the duration of the procedure. It can be tedious to seek a particular sequence of video frames within a long video record for manual annotation of a surgical highlight. Currently, annotation of surgical videos can be highly inefficient.

Herein are disclosed methods, apparatuses, systems, and microscopes which can reduce errors and/or tedious work in annotation of surgical videos.

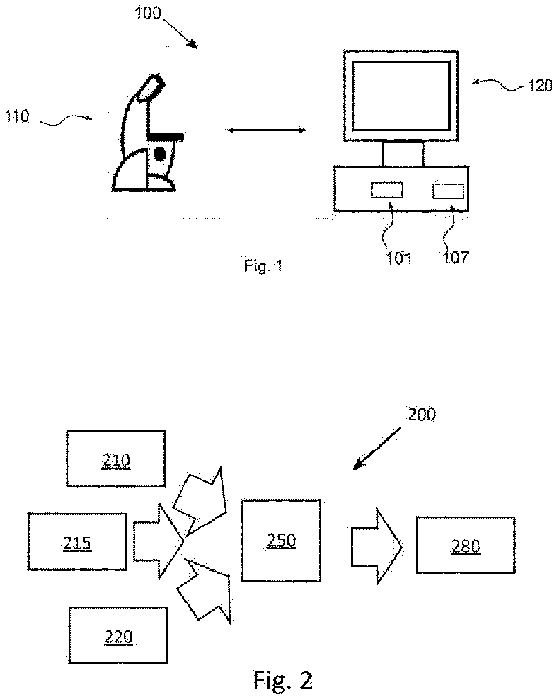

Some embodiments relate to a microscope comprising a system as described in connection with one or more of the Figs. 1 to 5. Alternatively, a microscope may be part of or connected to a system as described in connection with one or more of the Figs. 1 to 5.

Fig. 1 shows a schematic illustration of a system 100 configured to perform a method described herein. The system 100 comprises a microscope 110 and a computer system 120. The microscope 110 is configured to take images and is connected to the computer system 120. The computer system 120 is configured to execute at least a part of a method described herein. The computer system 120 may be configured to execute a machine learning algorithm. The computer system 120 and microscope 110 may be separate entities but can also be integrated together in one common housing. The computer system 120 may be part of a central processing system of the microscope 110 and/or the computer system 120 may be part of a subcomponent of the microscope 110, such as a sensor, an actor, a camera or an illumination unit, etc. of the microscope 110.

The computer system can include a processor 101. The computer system can include a memory 107. The processor 101 and/or memory 107 can be configured to operate the microscope and/or to perform the methods described herein, particularly annotating a video of a surgical procedure, including in real time during a recording of the surgery.

The computer system 120 may be a local computer device (e.g. personal computer, laptop, tablet computer or mobile phone) with one or more processors and one or more storage devices or may be a distributed computer system (e.g. a cloud computing system with one or more processors and one or more storage devices distributed at various locations, for example, at a local client and/or one or more remote server farms and/or data centers). The computer system 120 may comprise any circuit or combination of circuits. In one embodiment, the computer system 120 may include one or more processors which can be of any type. As used herein, processor may mean any type of computational circuit, such as but not limited to a microprocessor, a microcontroller, a complex instruction set computing (CISC) microprocessor, a reduced instruction set computing (RISC) microprocessor, a very long instruction word (VLIW) microprocessor, a graphics processor, a digital signal processor (DSP), multiple core processor, a field programmable gate array (FPGA), for example, of a microscope or a microscope component (e.g. camera) or any other type of processor or processing circuit. Other types of circuits that may be included in the computer system 120 may be a custom circuit, an application-specific integrated circuit (ASIC), or the like, such as, for example, one or more circuits (such as a communication circuit) for use in wireless devices like mobile telephones, tablet computers, laptop computers, two-way radios, and similar electronic systems. The computer system 120 may include one or more storage devices, which may include one or more memory elements suitable to the particular application, such as a main memory in the form of random access memory (RAM), one or more hard drives, and/or one or more drives that handle removable media such as compact disks (CD), flash memory cards, digital video disk (DVD), and the like. The computer system 120 may also include a display device, one or more speakers, and a keyboard and/or controller, which can include a mouse, trackball, touch screen, voice-recognition device, or any other device that permits a system user to input information into and receive information from the computer system 120.

Some or all of the method steps may be executed by (or using) a hardware apparatus, like for example, a processor, a microprocessor, a programmable computer or an electronic circuit. In some embodiments, some one or more of the most important method steps may be executed by such an apparatus.

Depending on certain implementation requirements, embodiments of the invention can be implemented in hardware or in software. The implementation can be performed using a non-transitory storage medium such as a digital storage medium, for example a floppy disc, a DVD, a Blu-Ray, a CD, a ROM, a PROM, and EPROM, an EEPROM or a FLASH memory, having electronically readable control signals stored thereon, which cooperate (or are capable of cooperating) with a programmable computer system such that the respective method is performed. Therefore, the digital storage medium may be computer readable.

Some embodiments according to the invention comprise a data carrier having electronically readable control signals, which are capable of cooperating with a programmable computer system, such that one of the methods described herein is performed.

Generally, embodiments of the present invention can be implemented as a computer program product with a program code, the program code being operative for performing one of the methods when the computer program product runs on a computer. The program code may, for example, be stored on a machine readable carrier.

Other embodiments comprise the computer program for performing one of the methods described herein, stored on a machine readable carrier.

In other words, an embodiment of the present invention is, therefore, a computer program having a program code for performing one of the methods described herein, when the computer program runs on a computer.

A further embodiment of the present invention is, therefore, a storage medium (or a data carrier, or a computer-readable medium) comprising, stored thereon, the computer program for performing one of the methods described herein when it is performed by a processor. The data carrier, the digital storage medium or the recorded medium are typically tangible and/or non-transitionary. A further embodiment of the present invention is an apparatus as described herein comprising a processor and the storage medium.

A further embodiment of the invention is, therefore, a data stream or a sequence of signals representing the computer program for performing one of the methods described herein. The data stream or the sequence of signals may, for example, be configured to be transferred via a data communication connection, for example, via the internet.

A further embodiment comprises a processing means, for example, a computer or a programmable logic device, configured to, or adapted to, perform one of the methods described herein.

A further embodiment comprises a computer having installed thereon the computer program for performing one of the methods described herein.

A further embodiment according to the invention comprises an apparatus or a system configured to transfer (for example, electronically or optically) a computer program for performing one of the methods described herein to a receiver. The receiver may, for example, be a computer, a mobile device, a memory device or the like. The apparatus or system may, for example, comprise a file server for transferring the computer program to the receiver.

In some embodiments, a programmable logic device (for example, a field programmable gate array) may be used to perform some or all of the functionalities of the methods described herein. In some embodiments, a field programmable gate array may cooperate with a microprocessor in order to perform one of the methods described herein. Generally, the methods are preferably performed by any hardware apparatus.

The system 100, microscope 110, and/or computer system 120 can be configured to annotate a video of a surgical procedure. The annotation(s) can be based on a command(s) executed during the surgical procedure, an instrument setting, and/or a video of the surgical procedure. Herein, a command can be an instrument command, such as a command that is processed/executed by the computer system 120, microscope 110, and/or other surgical instrument, particularly during the surgical procedure. Alternatively/additionally, the annotation(s) can be based at least partially on an instrument setting.

The annotation may be determined at least partially from the video. For example, a frame and/or sequence of frames of the video can be at least partially recognized, and an annotation associated with and/or added to the frame(s). For example, an object in the frame(s) is identified and the identification is used as at least a partial basis for an annotation. Alternatively/additionally, dictation can be used to generate and/or edit annotations. Language models trained using medical corpus and manually annotated medical records can also be used to generate annotations.

A command which can result in an annotation can be, for example, a command for determination of an optical setting of an optical surgical instrument, such as a microscope 110. There can be more than one command for determining optical setting(s), for example illumination intensity, focus, and/or magnification. Alternatively/additionally, the settings/commands can include a command for starting a record, such as starting a video capture. Commands used during surgery may alternatively/additionally be used to annotate the video. For example, commands (e.g. surgical commands) can include at least one of ultrasound activation, ultrasound deactivation, pump activation, injection, or suction.

The annotation can alternatively/additionally be determined based on one or more of an image parameter (such as a light level and/or average color), an object in the video (such as detection of a surgical instrument appearing within the video), and/or a movement (such as movement of an object like an anatomical feature or surgical instrument) within the video. The processor can be enabled to identify an object including at least one surgical instrument (e.g. a blade, or a phacoemulsifier) or at least one anatomical structure. Particular types of blades may be identifiable, such as a paracentesis blade, a keratome blade, and/or a cystotome blade.

The apparatus can also be configured to determine a time stamp associated with the annotation. The time stamp can be included in the annotation and/or associated with the annotation. An annotated video may include one or more annotations, each having associated time stamps. In replaying the annotated video, time stamps may allow a user to reach video content, at a time stamp, by selecting the associated annotation and/or time stamp.

The apparatus can include a memory that stores a library of annotations. The annotation, or at least one of a plurality of annotations, can be selected from the library.

In an embodiment that can be combined with any other embodiment described herein, a machine learning algorithm can be used to determine the annotation. For example, the machine learning algorithm can identify at least one of a command, a setting, an image parameter, an object, or a movement. Alternatively/additionally, the machine learning algorithm can classify at least one of the command, the image parameter, the object, or the movement.

The computer system 120 may be regarded as an embodiment of an apparatus for annotating a video, as described herein. The computer system 120 may include a means for machine learning, such as a processor configured to determine annotation(s), as described herein. Alternatively/additionally, the computer system may be configured to perform the method of annotating a video as described herein.

Fig. 2 illustrates a method of annotating a video. A command 210, instrument setting/parameter 215, and/or video 220 may be input for determining 250 an annotation for the video. Once the annotation is determined, the video is annotated 280 with the annotation. The method can repeat, *e.g.* at different times and/or frames of the video.

The method 200 can include determining 250 an annotation for a video. Determining 250 the annotation can be based on a command 210, *e.g.* executed during a surgical procedure recorded by the video, an instrument parameter/setting, and/or the video 220 itself.

A command 210 and/or the video 220 may be a form of input to a machine learning algorithm which determines 250 the annotation. Annotating 280 the video can be in several forms, such as embedding text in the video, adding text to accompany the video, and/or adding text to superimpose/overlay at least one frame of the video. Alternatively/additionally, the annotation may be in the form of a subtitle and/or caption. The video 220 may include images of anatomical features and/or surgical instruments, which can be identified/recognized. Such identification/recognition can be by machine learning algorithms.

As an example of a determination 250 of the annotation, a machine learning algorithm can determine a number of input characteristics. For example, the algorithm can detect at least one of: an illumination state and/or command, such as that LEDs are being used for illumination; a lens moving command; a lens magnification command, *e.g.* to detect the magnification; a start video recording command; or the presence of a blade, such as a paracentesis blade in the image. In an example, based on the determination of the illumination state, lens position/movement, magnification, and presence of a paracentesis blade, the algorithm can determine that the video is of a 'port incision' step, and annotate the video frame(s) accordingly. The determination of the annotation can be based on a command executed at or before the relevant frame of the video, optical setting(s), and/or identification of an object within the video. The determination of the annotation can include identifying a step of a surgical procedure, e.g. the port incision step. The annotation 280 can be by the selection of a corresponding annotation from a library, *e.g.* one that identifies the video frame(s) as corresponding to a step of a surgical procedure, such as a port incision step.

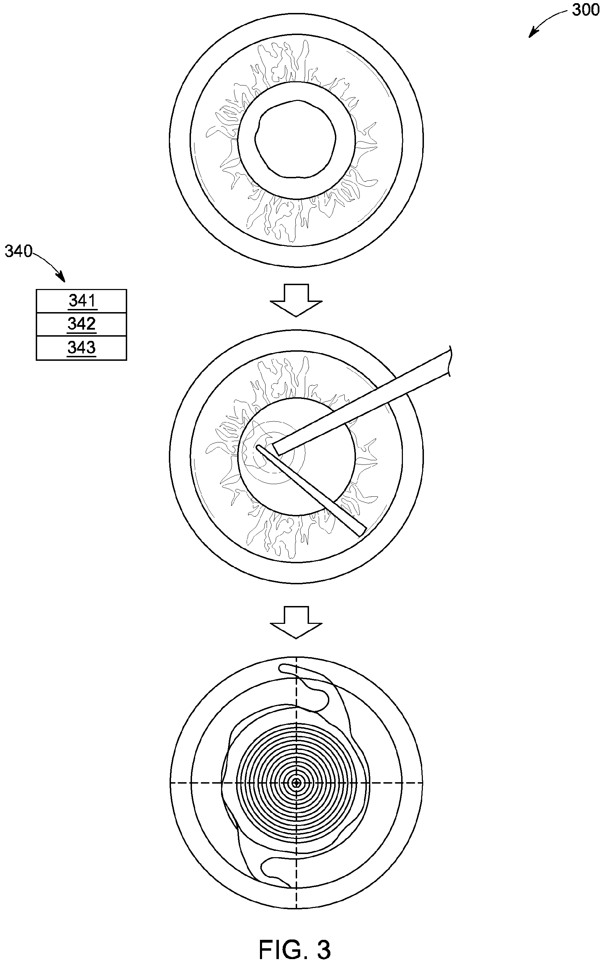

Fig. 3 illustrates a surgery workflow. Surgery workflows 300 can be routine and/or predictable. Surgeries can be deconstructed into different steps, which may include substeps. Surgeries can be deconstructed at a high level to include a few major steps, such as in the example of Fig. 3. Major steps may be deconstructed to provide more detailed steps (e.g. substeps) of the surgical procedure. Fig. 3 illustrates three steps, as an example. In step 1, the top frame of Fig. 3, access is made to a cataract. This can be done by making a few small incisions. In step 2, the middle frame of Fig. 3, a clouded lens may be broken up and/or removed. In step 3, a replacement can be made. In step 3 a new intraocular lens can be implanted.

Step 1 is an example of how an anatomical structure can appear in a frame of the video. The anatomical structure can be identified, such as by the machine learning algorithm, to aid in determining the annotation. For example, the lens capsule opening can be recognized in the frame of the video. The shape of the lens capsule opening can be irregular.

Step 2 is an example of how an object such as a surgical instrument(s) can appear in a frame of the video. The object(s) can be identified, such as by the machine learning algorithm, to aid in determining the annotation. Alternatively/additionally, surgical procedure may include execution of a command, such as executing ultrasound, which may be used to determine an annotation. Alternatively/additionally, breakup of a clouded lens may be determined in the surgical procedure, by a combination of determining activation of ultrasound (e.g. via command) and image analysis which recognizes the clouded lens, particularly being exposed to ultrasound. Step 2 can exemplify that a combination of commands and video/video analysis can be used as the basis for determining an annotation, e.g utilizing a machine learning algorithm. Step 3 can exemplify the recognition/identification of an implant, e.g. a lens, in a video frame(s) as being at least part of the basis of a determination of an annotation.

In an example, the annotations can be determined by selection from a library 340. The library may include a plurality of selections 341, 342, 343 for use as annotations. For example, the annotation for step 1 is selection 341 of the library 340. The annotation for step 2 can be selection 342 of the library. The annotation for step 3 can be selection 343 of the library. The selection(s) 341, 342, and/or 343 annotations can be editable by a user.

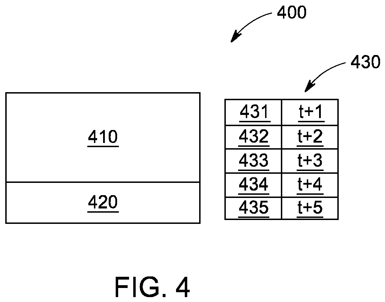

Fig. 4 illustrates an annotated video. The annotated video 400 of Fig. 4 shows a frame 410 of the video 400 with an associatd annotation 420. The video 400 can include multiple annotations 431, 432, 433, 434, 435. The annotations 431, 432, 433, 434, 435 can be associated with time stamps. Each annotation 420, 431, 432, 433, 434, 435 can be optionally displayed with video frames, of the video 400, that are associated with the annotations and/or time stamps. For example, the video 400 has the frame 410 at time t which has the associated annotation 420. At time t+1, the video has a frame associated with annotation 431, which can be displayed at time t+1. Each annotation 432, 433, 434, 435 can have respective associated time stamps t+2, t+3, t+4, and t+5, e.g. times at which the respective annotations can be displayed.

The annotation can occur during the collection of the video and/or during the surgical procedure, *e.g.* in real-time. Time stamps and annotations can be determined, and possibly displayed in menu format, e.g. as shown with the table of 430 of Fig. 4 which associates annotations 431 through 435 with times t+1 through t+5, respectively. The annotated video can include data such as metadata, e.g. for easily editing annotations, including time stamps and annotations. A user may select an annotation and/or time stamp, while viewing the video, to skip to the frame(s) of the video associated with the time stamp and/or annotation.

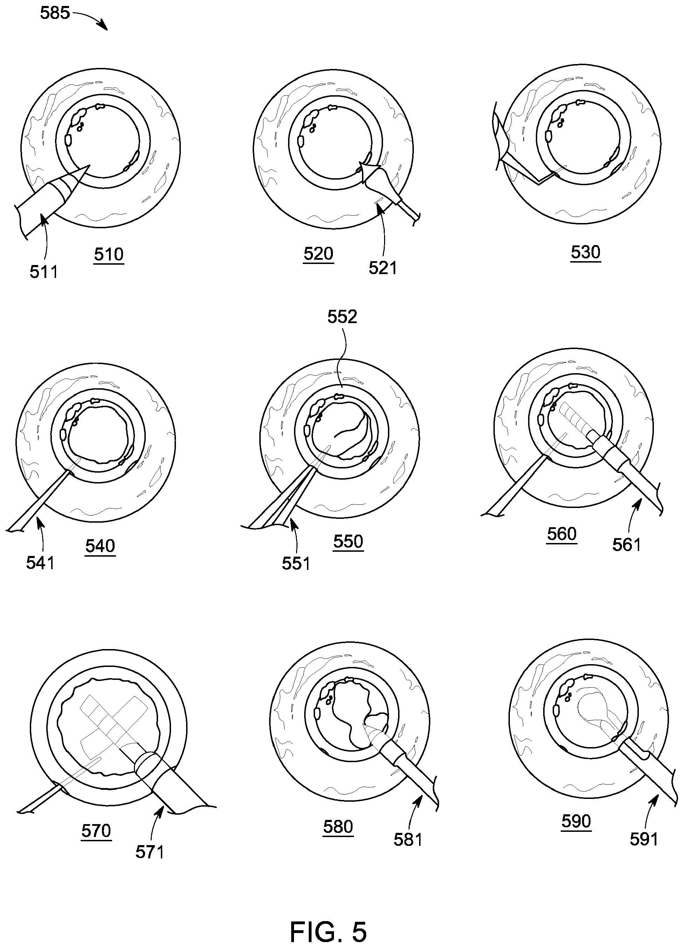

Fig. 5 illustrates a surgical procedure. The procedure is represented as a series of frames of a video in Fig. 5. A video recording of a surgical procedure can include multiple annotations 510 - 590, such as at different times of the recording. Each annotation 510-590 can be determined, as described herein, and included in the video 400. In the example of Fig. 5, a cataract surgery is depicted. It is possible that the determination of the annotation(s) 510 - 590 can be based at least in part on user-provided information, for example, identification of the type of surgery, e.g. cataract surgery.

The first annotation 510 can refer to a port incision. The port incision can be determined on the basis of at least one of: a command that the light is on, command(s) regarding focusing, command(s)regarding magnification, command(s) regarding the start of the recording, or identification of a paracentesis blade 511 in the video (e.g. in a frame or series of frames).

Like any annotation that follows a previous annotation (e.g. the first annotation 510), the second annotation 520 can be determined at least partially on the basis that the surgical procedure step is following the previous annotation(s)/step(s).

The second annotation 520, as in the example of a cataract surgery, can refer to a second incision. The second incision 520 can be determined at least partly on identification of an object in the video, e.g. a keratome blade 521.

The third annotation 530, as in the example of a cataract surgery of Fig. 5, can be determined to be application of a viscoelastic material (e.g. viscoat). The determination of an annotation, as in annotation 530, can be based at least partially on a pump command, and/or an injection command, such as corresponding to the injection of viscoelastic material. Alternatively/additionally, the determination can be based at least partially on an injection of anesthetic, which may be in the form of a pump command.

The fourth annotation 540 can be determined to be continuous curvilinear capsulorhesis. The fourth annotation 540 can be based at least partially on detecting an object such as a cystotome blade 541 and/or a pump command such as the injection of anesthetic.

The fifth annotation 550 can be determined to be flap creation. The determination of flap creation can be based at least partially on detecting an object such as a flap 552 and/or blade (e.g. a cystotome blade 551), and/or detecting a motion, such as the motion of peeling away the flap 552.

The sixth annotation 560 can be determined to be phacoemulsification. The determination of flap creation can be based at least partially on detecting an object such as a phacoemulsifier 561; determination of a command for ultrasonification; and/or motion detection.

The seventh annotation 570 can be determined to be suction. The determination of suction can be based on detection of an object, such as a phacoemulsifier 571, detection of a suction command, and/or motion of suction.

The eighth annotation 580 can be determined to be irrigation/aspiration. The determination of irrigation/aspiration can be based at least partially on object detection such as an aspiration hand piece 581; a motion detection such as a cleaning motion, and/or a command such as an illumination command, particularly red-reflex illumination.

The ninth annotation 590 can be determined to be an intraocular lens insertion. The determination of intraocular lens insertion can be based at least partially on detecting an object such as a lens injector 591, and/or a motion detection such as adjustments of the position of the lens.

In an embodiment that can be combined with any other embodiment described herein, the relative positions and/or relative movements of two or more objects identified in the video can be a partial or greater basis for determining an annotation.

Fig. 5 also illustrates an annotation insertion 585. For example, a user may insert an annotation in the video. Alternatively/additionally, a machine learning algorithm may determine an annotation insertion 585, or suggest an annotation insertion 585. For example, the procedure may include an offline activity, such as lens preparation. An offline activity can be identified by a period of inactivity in the video and/or a pause in recording, for example. Alternatively/additionally, an offline activity can be identified based at least partially on detecting no illumination, no illumination command, a lack of detected motion in the video, and/or a movement of the surgical instrument, such as movement of a surgical microscope up from the surgical site. The annotation insertion 585 can be determined to be a lens preparation for example, based at least partially on the neighboring annotations 580, 590, which may be known to be correlated with lens preparation being therebetween.

Objects, such as paracentesis blades and viscoelastic material, can appear during surgeries. Object identification and/or classification, particularly in combination with determined motions associated with user-performed surgery steps, may suggest/determine annotations. For example, when annotations are determined by machine learning algorithm, they may be subsequently edited by the surgeon/user, *e.g.* to confirm accuracy. Activities that happen during surgeries, such as peeling flap away during a flap creation step in standard cataract surgery, are often highlights of the surgery which are particularly desirable to have associated annotations. Convolutional neural networks trained specifically for medical object detection and/or activity classification (e.g. movement detection), optionally combined with language models which can be trained on medical corpus can determine/suggest annotation for surgical videos.

System commands issued during the surgeries, such as when the user adjusts the system configuration, may also be at least the partial basis for the determination of an annotation, and/or may suggest a step of a surgical process. Combined with a trained neural network, a more detailed annotation can be determine, which can possibly document the medical surgery actions and/or configurational changes of the system.

When a user performs surgery, system commands can be issued during the process, such as controlling lights, zoom magnifications, etc. Such system commands can be captured to generate information regarding the detailed system configuration during certain medical steps. The system commands and/or system state can be included in the annotations, such as alternative annotations which are accessible when viewing the video if the viewer is interested in the system settings during the procedure. The medical objects, blades, viscoelastic material, etc. which may appear in the video during the surgery, can also be included in the annotation, such as identified and highlighted as overlay in the frame(s), such as by trained convolutional neural networks. Activities performed by the user, such as incision or peeling away flap, can be identified and classified as well, such as using a machine learning algorithm to correlate motions captured in the video with suitable annotations, e.g. annotations which explain steps of surgical procedures.

Furthermore, it is possible to use language models trained using medical corpus and/or manually annotated medical records to determine annotations, such as descriptions of the object(s) which appear in the frame(s) of the video, and/or the activities captured in the video of the surgery.

For example, system configuration changes, highlighted objects that appear in the video, and identified activities (such as motions/steps of a surgical procedure) can be combined to generate annotations. The annotations may appear in the video directly, for example, such as in an overlaid/superimposed format, as highlighting the action/object seen in frame, as subtitles. Annotations may also include supplemental material associated with the video, such as instrument parameters, settings, and/or instrument commands. Such annotations may greatly aid in describing the surgical procedure, for example, for pedagogical purposes. For example, annotation(s) can be embedded into the video stream as stopping points and/or time stamps. Users can jump to time stamped frame(s) of the video, e.g. to rapidly locate and review desired content. Alternatively/additionally, annotations can be in the form of subtitles, e.g. in the format of srt (subRip), ssa (substation alpha), and/or ass (Advanced SubStation Alpha). Alternatively/additionally the annotations can be embedded into the video/video container (e.g. MP4, MKV, etc).

Embodiments may be based on using a machine-learning model and/or machine-learning algorithm. Machine learning may refer to algorithms and statistical models that computer systems may use to perform a specific task without using explicit instructions, instead relying on models and inference. For example, in machine-learning, instead of a rule-based transformation of data, a transformation of data may be used, that is inferred from an analysis of historical and/or training data. For example, the content of images may be analyzed using a machine-learning model or using a machine-learning algorithm. In order for the machine-learning model to analyze the content of an image, the machine-learning model may be trained using training images as input and training content information as output. By training the machine-learning model with a large number of training images and/or training sequences (e.g. words or sentences) and associated training content information (e.g. labels or annotations), the machine-learning model "learns" to recognize the content of the images, so the content of images that are not included in the training data can be recognized using the machine-learning model. The same principle may be used for other kinds of sensor data as well: By training a machine-learning model using training sensor data and a desired output, the machine-learning model "learns" a transformation between the sensor data and the output, which can be used to provide an output based on non-training sensor data provided to the machine-learning model. The provided data (e.g. sensor data, meta data and/or image data) may be preprocessed to obtain a feature vector, which is used as input to the machine-learning model.

Machine-learning models may be trained using training input data. The examples specified above use a training method called "supervised learning". In supervised learning, the machine-learning model is trained using a plurality of training samples, wherein each sample may comprise a plurality of input data values, and a plurality of desired output values, i.e. each training sample is associated with a desired output value. By specifying both training samples and desired output values, the machine-learning model "learns" which output value to provide based on an input sample that is similar to the samples provided during the training. Apart from supervised learning, semi-supervised learning may be used. In semi-supervised learning, some of the training samples lack a corresponding desired output value. Supervised learning may be based on a supervised learning algorithm (e.g. a classification algorithm, a regression algorithm or a similarity learning algorithm. Classification algorithms may be used when the outputs are restricted to a limited set of values (categorical variables), i.e. the input is classified to one of the limited set of values. Regression algorithms may be used when the outputs may have any numerical value (within a range). Similarity learning algorithms may be similar to both classification and regression algorithms but are based on learning from examples using a similarity function that measures how similar or related two objects are. Apart from supervised or semi-supervised learning, unsupervised learning may be used to train the machine-learning model. In unsupervised learning, (only) input data might be supplied and an unsupervised learning algorithm may be used to find structure in the input data (e.g. by grouping or clustering the input data, finding commonalities in the data). Clustering is the assignment of input data comprising a plurality of input values into subsets (clusters) so that input values within the same cluster are similar according to one or more (pre-defined) similarity criteria, while being dissimilar to input values that are included in other clusters.

Reinforcement learning is a third group of machine-learning algorithms. In other words, reinforcement learning may be used to train the machine-learning model. In reinforcement learning, one or more software actors (called "software agents") are trained to take actions in an environment. Based on the taken actions, a reward is calculated. Reinforcement learning is based on training the one or more software agents to choose the actions such, that the cumulative reward is increased, leading to software agents that become better at the task they are given (as evidenced by increasing rewards).

Furthermore, some techniques may be applied to some of the machine-learning algorithms. For example, feature learning may be used. In other words, the machine-learning model may at least partially be trained using feature learning, and/or the machine-learning algorithm may comprise a feature learning component. Feature learning algorithms, which may be called representation learning algorithms, may preserve the information in their input but also transform it in a way that makes it useful, often as a pre-processing step before performing classification or predictions. Feature learning may be based on principal components analysis or cluster analysis, for example.

In some examples, anomaly detection (i.e. outlier detection) may be used, which is aimed at providing an identification of input values that raise suspicions by differing significantly from the majority of input or training data. In other words, the machine-learning model may at least partially be trained using anomaly detection, and/or the machine-learning algorithm may comprise an anomaly detection component.

In some examples, the machine-learning algorithm may use a decision tree as a predictive model. In other words, the machine-learning model may be based on a decision tree. In a decision tree, observations about an item (e.g. a set of input values) may be represented by the branches of the decision tree, and an output value corresponding to the item may be represented by the leaves of the decision tree. Decision trees may support both discrete values and continuous values as output values. If discrete values are used, the decision tree may be denoted a classification tree, if continuous values are used, the decision tree may be denoted a regression tree.

Association rules are a further technique that may be used in machine-learning algorithms. In other words, the machine-learning model may be based on one or more association rules. Association rules are created by identifying relationships between variables in large amounts of data. The machine-learning algorithm may identify and/or utilize one or more relational rules that represent the knowledge that is derived from the data. The rules may e.g. be used to store, manipulate or apply the knowledge.

Machine-learning algorithms are usually based on a machine-learning model. In other words, the term "machine-learning algorithm" may denote a set of instructions that may be used to create, train or use a machine-learning model. The term "machine-learning model" may denote a data structure and/or set of rules that represents the learned knowledge (e.g. based on the training performed by the machine-learning algorithm). In embodiments, the usage of a machine-learning algorithm may imply the usage of an underlying machine-learning model (or of a plurality of underlying machine-learning models). The usage of a machine-learning model may imply that the machine-learning model and/or the data structure/set of rules that is the machine-learning model is trained by a machine-learning algorithm.

For example, the machine-learning model may be an artificial neural network (ANN). ANNs are systems that are inspired by biological neural networks, such as can be found in a retina or a brain. ANNs comprise a plurality of interconnected nodes and a plurality of connections, so-called edges, between the nodes. There are usually three types of nodes, input nodes that receiving input values, hidden nodes that are (only) connected to other nodes, and output nodes that provide output values. Each node may represent an artificial neuron. Each edge may transmit information, from one node to another. The output of a node may be defined as a (non-linear) function of its inputs (e.g. of the sum of its inputs). The inputs of a node may be used in the function based on a "weight" of the edge or of the node that provides the input. The weight of nodes and/or of edges may be adjusted in the learning process. In other words, the training of an artificial neural network may comprise adjusting the weights of the nodes and/or edges of the artificial neural network, i.e. to achieve a desired output for a given input.

Alternatively, the machine-learning model may be a support vector machine, a random forest model or a gradient boosting model. Support vector machines (i.e. support vector networks) are supervised learning models with associated learning algorithms that may be used to analyze data (e.g. in classification or regression analysis). Support vector machines may be trained by providing an input with a plurality of training input values that belong to one of two categories. The support vector machine may be trained to assign a new input value to one of the two categories. Alternatively, the machine-learning model may be a Bayesian network, which is a probabilistic directed acyclic graphical model. A Bayesian network may represent a set of random variables and their conditional dependencies using a directed acyclic graph. Alternatively, the machine-learning model may be based on a genetic algorithm, which is a search algorithm and heuristic technique that mimics the process of natural selection.

It is particularly contemplated that the method of annotation and apparatus disclosed herein can possibly dynamically improve the accuracy and reduce error of annotations, including when the data for the machine learning algorithm is expanded. Training data may be alternatively/additionally used initially and/or added to the memory accessible to the machine learning algorithm at later times. Data, particularly additional data which may be available to the machine learning algorithm after the apparatus is in operation, may further expand the possible annotations (e.g. the library 340 of possible annotations may increase) and/or improve accuracy of the determination/selection of the annotation.

Examples described herein are for illustration. The invention is defined by the appended claims and their equivalents.

**List of reference Signs**

| | |
|---|---|
| system | 100 |
| processor | 101 |
| memory | 107 |
| microscope | 110 |
| computer system | 120 |
| method of annotating | 200 |
| command | 210 |
| instrument parameter/setting | 215 |
| video | 220 |
| determining annotation | 250 |
| annotating the video | 280 |
| surgery workflow | 300 |
| library | 340 |
| first selection | 341 |
| second selection | 342 |
| third selection | 343 |
| annotated video | 400 |
| frame | 410 |
| annotation | 420 |
| table of annotations | 430 |
| first annotation | 431 |
| second annotation | 432 |
| third annotation | 433 |
| fourth annotation | 434 |
| fifth annotation | 435 |
| first annotation | 510 |
| paracentesis blade | 511 |
| second annotation | 520 |
| keratome blade | 521 |
| third annotation | 530 |
| fourth annotation | 540 |
| cystome blade | 541 |
| fifth annotation | 550 |
| cystome blade | 551 |
| flap | 552 |
| sixth annotation | 560 |
| phacoemulsifier | 561 |
| seventh annotation | 570 |
| phacoemulsifier | 571 |
| eighth annotation | 580 |
| aspiration hand piece | 581 |
| annotation insertion | 585 |
| ninth annotation | 590 |
| lens injector | 591 |

## Claims

1. An apparatus (120), configured to:
annotate (280) a video (220) of a surgical procedure based on at least one of:
a command (210) executed during the surgical procedure, an instrument setting, or the video (220).

2. The apparatus (120) of claim 1, wherein
the command (210) is for setting at least one of a plurality of optical settings of an optical instrument.

3. The apparatus (120) of claim 2, wherein
the plurality of optical settings includes at least one of illumination intensity, focus, illumination source, color filter, or magnification.

4. The apparatus (120) of any preceding claim, wherein
the command (110) is at least one of a plurality of surgical commands of a surgical instrument (120).

5. The apparatus (120) of claim 4, wherein
the plurality of surgical commands are for at least two of ultrasound activation, ultrasound deactivation, pump activation, pump deactivation, injection, or suction.

6. The apparatus (120) of any preceding claim,
configured to determine the annotation based on
at least one of: an image parameter of the video (120), an object (511) in the video, relative positions of at least two objects (551, 552) in the video, or a movement in the video.

7. The apparatus (120) of claim 6, wherein
the object is at least one of a plurality of identifiable objects including;
at least one surgical instrument (511) or at least one anatomical structure (552).

8. The apparatus (120) of claim 7, wherein
the at least one surgical instrument is a blade (521) or a phacoemulsifier (561).

9. The apparatus (120) of any preceding claim, configured to determine
a time stamp (t+1) associated with the annotation (431).

10. The apparatus (120) of any preceding claim, further comprising
a memory (107), wherein
the annotation (420) is selected from a library (340) stored in the memory (107).

11. The apparatus (120) of any preceding claim, wherein
the apparatus is configured such that the video (120) is annotated with a plurality of annotations (431,432, 433), each annotation having an associated time stamp (t+1, t+2, t+3).

12. The apparatus (120) of any preceding claim, configured to determine (250) the annotation (420) by a machine learning algorithm.

13. The apparatus (120) of claim 12, wherein
the machine learning algorithm is configured to at least one of:
identify at least one of the command (110), the image parameter, an object, or
a movement; or
classify at least one of the command (110), the image parameter, the object, or
the movement.

14. A method of annotating a video (120) of a surgical procedure, comprising:
determining (250) an annotation for a video (120) of a surgical procedure, and
annotating (280) the video (120) with the annotation, wherein
determining the annotation is based on at least one of:
a command (210) executed during a procedure recorded by the video (120),
an instrument setting, or
a determination of at least one of: an image parameter of the video (120), an object in the video (120), or a movement in the video.

15. A computer program with a program code for annotating a video (120) of a surgical procedure, the annotating being based on at least one of:
a command (110) executed during the surgical procedure, an instrument parameter, or
the video (120).
